# EUROPEAN PATENT APPLICATION

(11) **EP 4 446 432 A1**
(43) Date of publication of application: **16.10.2024**
(21) Application number: 22904697.4
(22) Date of filing: 08.12.2022
(51) Int. Cl.: C12Q 1/6848, C12Q 1/6827, C12Q 1/6862, C12Q 1/70

(54) **METHOD FOR GENERATING SINGLE STRAND AND METHOD FOR DETECTING MUTATION USING SAME**

(30) Priority: 09.12.2021 KR 20210176103; 13.04.2022 KR 20220045702
(71) Applicant: POSTECH Research and Business Development Foundation, Pohang-si, Gyeongsangbuk-do 37673 (KR)
(72) Inventor: LEE, Jeong Wook, Pohang-si, Gyeongsangbuk-do 37673 (KR); WOO, Chang Ha, Pohang-si, Gyeongsangbuk-do 37673 (KR); LEE, Jeongmin, Pohang-si, Gyeongsangbuk-do 37673 (KR); SUNG, Doeon, Pohang-si, Gyeongsangbuk-do 37673 (KR)
(74) Representative: Altmann Stößel Dick Patentanwälte PartG mbB
(86) International application number: PCT/KR2022/019970
(87) International publication number: WO 2023/106868

(57) **Abstract**

The present invention relates to a method for generating a single-stranded region for detecting genetic mutation and a method for detecting genetic mutation using same. A detection method according to the present invention can detect sequence-specific nucleic acids and detect mutations at the single nucleotide polymorphism (SNP) discrimination level, thereby allowing for the accurate and sensitive discrimination of mutations in a short time. Thus, the present invention provides an effective mutation detection method that can respond not only to current SARS-CoV-2 variants but also generally to specific diseases or related mutations in the future.

## Description

### [Technical Field]

The present disclosure relates to a method for generating a single-stranded region for detecting genetic mutations and a method for detecting genetic mutations using the same. More specifically, the present disclosure relates to a method for generating a single-stranded region from PCR amplicons and a method for detecting sequence-specific nucleic acids and detecting mutations at the discrimination level of single nucleotide polymorphism (SNP) using the same.

### [Background Art]

Due to the increase in global trade and travel, global infectious diseases have become more frequent and are spreading rapidly. Since many life-threatening infectious diseases have signs and symptoms similar to those of a cold or flu-like syndrome, technology for early and accurate diagnosis of these diseases is essential for providing appropriate medical services. In addition, in terms of preventing the spread of infectious diseases, it is also necessary to develop technology capable of quickly determining infection in the field.

Since the COVID-19 pandemic began, many virus variants have emerged around the world. The SARS-CoV-2 Interagency Group (SIG) has implemented a classification of variants into four classes, and the classification system and methodology are outlined as follows.
- Variants being monitored (VBM, variants that are no longer detected in the United States or are present at extremely low levels and do not pose a significant or imminent risk to public health): Alpha (B.1.1.7 and Q lineages), Beta (B.1.351 and descendent lineages), Gamma (P.1 and descendent lineages), Epsilon (B.1.427, B.1.429), Eta (B.1.525), Iota (B.1.526), Kappa (B.1.617.1), Mu (B.1.621, B.1.621.1), Zeta (P.2).
- Variants of interest (VOI, variants having observation of specific genetic markers associated with changes in receptor binding, attenuation the neutralizing response of antibodies generated by previous infection or vaccination, reduction of therapeutic efficacy, potential diagnostic implications, or a predicted increase in infectivity or disease severity).
- Variants of concern (VOC, variants having evidence for greater transmissibility, more severe disease (e.g., increased hospitalization and death), significant reduction in neutralizing antibody responses generated by previous infection or vaccination, reduced effectiveness of treatments or vaccines, and failure to detect diagnostics): Delta (B.1.617.2 and AY lineages), Omicron (B.1.1.529).
- Variants of high consequence (VOHC, variants having clear evidence of significantly lower preventative or medical countermeasures (MCM) effectiveness compared to previously prevalent variants).

Mutant viruses designated as variants of concern or variants of high consequence need to be managed separately. However, among the currently developed mutation detection methods, technology capable of easily and quickly distinguishing the above mutations without expensive equipment is underdeveloped.

In particular, since molecular diagnostic assay using nucleic acids, which is currently the most widely used for diagnosing infectious diseases, has greater sensitivity than existing diagnostic methods, it is possible to detect specific genes for the purpose of disease prevention, to conduct screening tests to prevent infectious diseases, early confirmation, and rapid response; nevertheless, most molecular diagnostic technologies using nucleic acids are hindered by the drawback of requiring thermocycler equipment and specialized personnel capable of proficiently operating the machine.

Meanwhile, loop-mediated isothermal amplification assay (LAMP) is an alternative to thermal cycling-based amplification method, which is similar to the existing PCR method, but the isothermal amplification method allows annealing and extension at a constant temperature, while the existing PCR method involves temperature changes through three phases: denaturation, annealing, and extension. This is made possible by using Bst. DNA polymerase instead of using Taq. DNA polymerase used in conventional PCR. Unlike the Taq. DNA polymerase commonly employed in conventional PCR, Bst. DNA polymerase has the characteristics of a S',3' exonuclease, enabling to perform annealing and extension on the double helix structure of DNA at a temperature closely aligned with the Tm value without denaturation of the DNA double helix structure by heat.

The isothermal amplification method has the advantage of allowing diagnosis to be performed without special laboratory techniques, by amplifying and diagnosing trace amounts of infectious agents (such as viruses or bacteria) without reliance on expensive equipment due to high amplification efficiency and sensitivity. However, the complex composition of the mixture used in the isothermal amplification method often makes diagnosis of the infectious agent difficult.

Meanwhile, nucleotide sequence polymorphism refers to a mutation in nucleotide sequence that occurs at a frequency greater than 1%, of which 90% are single nucleotide polymorphisms (SNPs). Human genetic sequences are 99.9% identical, and SNPs occur approximately once every 250 to 1,000 bp. In the human genome, there are about 2 million SNPs, of which about 21,000 exist in genes.

SNPs play an important role not only in DNA sequence analysis, but also as markers capable of identifying genes that cause diseases such as cancer, asthma, and diabetes, or determining the relationship with a specific disease. Accordingly, SNP analysis is used to identify an individual's susceptibility to a specific disease in humans, which can be helpful in preventing congenital diseases caused by mutations in nucleotide sequences or in treating diseases that have developed.

Under this background, the present inventors have completed the present disclosure by developing not only a method for detecting and diagnosing mutant viruses from PCR amplicons very effectively and accurately in a short period of time without securing expensive equipment and skilled personnel, but also a method for detecting mutations at the discrimination level of single nucleotide polymorphism (SNP) and diagnosing diseases based on the results.

### [Disclosure]

### [Technical Problem]

An object of the present invention is to provide a composition for detecting a target nucleic acid comprising: an isothermal one-pot reaction SENSR probe set for detecting a target nucleic acid including a first probe and a second probe; and at least one nucleic acid for interfering with double strand recombination of target gene.

Another object of the present invention is to provide a composition for detecting a target gene comprising: an isothermal one-pot reaction SENSR first probe set for detecting a target nucleic acid, including a first probe and a second probe; an isothermal one-pot reaction SENSR second probe set for detecting a target nucleic acid mutation, including a third probe and a fourth probe; and at least one nucleic acid for interfering with double strand recombination of target gene.

Still another object of the present invention is to provide a kit for detecting a target gene comprising: the composition described above; a ligation agent, a polymerase, and an isothermal one-pot reaction buffer.

Still another object of the present invention is to provide a method for detecting a target gene comprising: (a) separating a double strand of the target gene at a high temperature of 80 to 100°C; (b) cooling a reaction product in (a) to an isothermal temperature of 15 to 50°C and then performing an isothermal one-pot reaction by adding the above-described composition; and (c) detecting signal generation.

Still another object of the present invention is to provide a method for providing information for single nucleotide polymorphism (SNP)-based disease diagnosis, comprising: (a) separating a double strand of a target gene at a high temperature of 80 to 100°C; (b) cooling a reaction product in (a) to an isothermal temperature of 15 to 50°C and then performing an isothermal one-pot reaction by adding the composition according to claim 8; (c) detecting signal generation; and (d) determining the onset of a genetic disease when a mutation at the level of single nucleotide polymorphism (SNP) in the gene is confirmed.

### [Technical Solution]

In the following specification, description of overlapping content will be omitted to prevent any potential confusion arising from redundancy. In other words, the content of the invention is not limited to the following content; rather, it should be construed in accordance with the comprehensive content of the invention.

Further, terms used herein are merely used for illustration purposes, which should not be construed as limiting the present invention. Singular expressions include plural expressions unless the context clearly indicates otherwise. In the present specification, terms such as "comprise" or "have" are intended to designate the presence of features, numbers, steps, operations, components, parts, or combinations thereof described in the specification and it should not be understood as precluding the possibility of the presence or addition of one or more other features, numbers, steps, operations, components, parts, or combinations thereof.

In addition, unless defined otherwise, all terms used herein, including technical or scientific terms, have the same meaning as commonly understood by one of ordinary skill in the art to which the embodiments belong. Terms such as those defined in commonly used dictionaries should be interpreted as having a meaning consistent with the meaning in the context of the related art, and unless explicitly defined in the present application, it is not to be construed in an idealized or overly formal sense.

The present invention provides a composition for detecting a target nucleic acid comprising: an isothermal one-pot reaction SENSR probe set for detecting a target nucleic acid including a first probe and a second probe; and at least one nucleic acid for interfering with double strand recombination of target gene.

To provide a more elaborate description of the composition for detecting a target nucleic acid of the present invention, the present invention may comprise an isothermal one-pot reaction SENSR probe set for detecting a target nucleic acid including a first probe and a second probe; and at least one nucleic acid for interfering with double strand recombination of target gene, thereby detecting the target nucleic acid with high specificity using the same.

The target nucleic acid may be a nucleic acid of any origin as long as it is a nucleic acid of a gene to be detected, but may be, for example, any one selected from genes of viruses, harmful bacteria, or animals, including humans.

In this case, the genes of animals, including humans, may be the nucleic acids themselves of the genes or single nucleotide polymorphism (SNP) genes of animals.

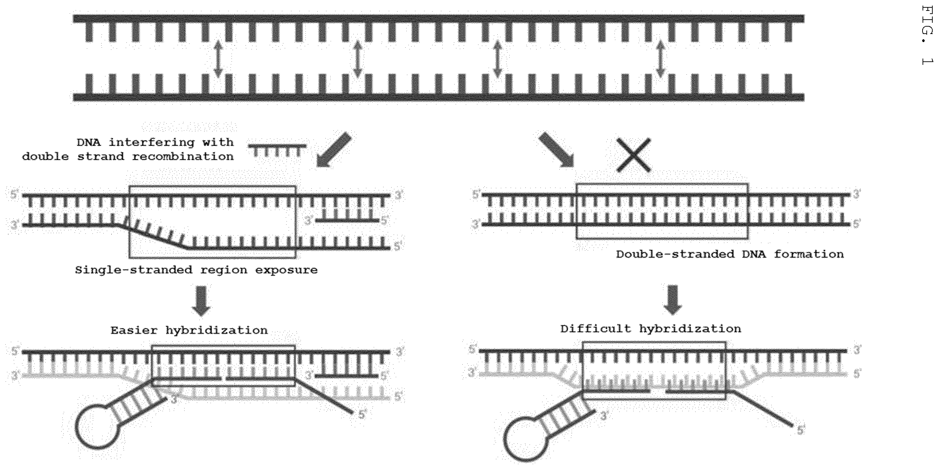

The principle of the composition comprising a probe set for isothermal one-pot reaction (SENSR probe set) and at least one nucleic acid for interfering with double strand recombination of target gene according to the present invention is shown in FIG. 1 as a schematic diagram thereof, and a more detailed description of the present invention is as follows.

The product intended for detection in the present invention corresponds to a nucleic acid sequence forming a double bond. As long as it is the nucleic acid sequence forming a double bond, any sequence from any origin may be used as a target sequence for detection in the present invention.

For example, according to an embodiment of the present invention, single-stranded regions are generated by using amplified DNA fragments (PCR amplicons) of a PCR product, and the regions are used for the purpose of sequence-specifically detecting nucleic acids and for the purpose of detecting the presence or absence of the mutation in the corresponding region.

The mutation may be any one selected from mutations in genes of, for example, viruses, harmful bacteria, and animals, including humans.

Here, the mutations in genes of animals, including humans, may be, for example, mutations in single nucleotide polymorphism (SNP) genes.

In the present invention, the principle of generating a single-stranded region for a double-stranded nucleic acid sequence is as follows.

In general, nucleic acid sequences (e.g., experimentally, PCR amplicons) tend to form a double-stranded structure in which single-strands with complementary sequences are combined. This double-stranded structure is very stable, but when the temperature of the nucleic acid sequence forming the double-stranded structure is raised 80°C or more, which is the denaturation temperature, the complementary single strands are separated from each other and may exist in a single-stranded state. When the temperature is lowered below the melting temperature, the nucleic acid sequence returns to a stable double-stranded structure.

Here, within the nucleic acid sequence forming a double bond, when there are single-stranded nucleic acid (DNA/RNA) fragments that are complementary to the single strand but are shorter in length than the nucleic acid sequence forming a double bond, and the temperature is lowered in the presence of these fragments, the short-length single-stranded nucleic acids (DNAs/RNAs) may be hybridized to interfere with the reformation of the double-stranded structure.

The single-stranded region formation process of the present invention is applicable to various types of nucleic acid sequences forming double bonds. For example, the process may be used for nucleic acid sequences forming double bonds isolated from any sample. In addition, it is also applicable not only to PCR amplicons that amplify DNA, but also to PCR amplicons obtained by converting RNA to DNA in a reverse-transcriptase reaction. It can also be applied to various isothermal amplification products as well as amplification products using thermocyclers such as PCR instruments.

In particular, the present invention found that the probe hybridization region may be more effectively exposed when DNAs for interfering with complementary double strand recombination are used together on the upstream and downstream sides of the target sequence. This may allow more SENSR probes to hybridize to the probe hybridization region and improve the specificity of the mutation discrimination reaction because the intensity of the fluorescence signal is more clearly different depending on the presence or absence of the mutation.

Meanwhile, the "SENSR assay" according to the present invention is a molecular diagnostic assay capable of determining the presence of a target sequence in a reaction product by measuring the degree of fluorescence. The SENSR reaction product consists of two probes capable of hybridizing to a single-stranded target sequence. In each probe, the T7 initiator sequence is placed at the top of the upstream probe, and the RNA aptamer template sequence is placed at the bottom of the downstream probe.

These two probes hybridize to the target sequence only when the target sequence is present and are located in close proximity to each other. When a ligation reaction occurs in two adjacent probes, the two probes are linked into a single strand of probe. When the linked probe is subjected to transcription by T7 RNA polymerase, an RNA aptamer is generated as a result, and a fluorescent signal is generated by the generated RNA aptamer.

This structure enables the probe set of the present invention to become a probe that exhibits a high detection effect in a short time under isothermal one-pot reaction conditions as a unified step.

More specifically, the probe design of the present invention allows two single-stranded DNA probes to expose the target recognition sequence, causing hybridization of the target RNA/DNA and the probe set at isothermal (e.g., a temperature at which the enzyme can act) conditions. Hybridization sequences are designed to maximize hybridization to the target RNA/DNA while minimizing the formation of any other structures. An efficient hybridization process between the probe and target RNA/DNA allows for high sensitivity during isothermal reactions.

The promoter probe is designed to form a stem-loop structure, and the stem portion forms a double-stranded RNA polymerase promoter sequence to initiate a transcription process using RNA polymerase. Since the double-stranded RNA polymerase promoter portion is physically linked by the loop sequence, the probability that the double-stranded promoter is formed into a functional form is higher than when the double-stranded promoter is not linked by the loop sequence. Therefore, the self-assembled promoter sequence in which the hairpin structure is formed in the promoter probe may effectively promote the hybridization process and the subsequent transcription process.

The reporter probe is designed to include an aptamer sequence as a reporter, and the final product may be identified by the aptamer that generates a signal in response to a specific substance.

In the present invention, "aptamer" used as a reporter is a single-stranded nucleic acid (DNA, RNA or modified nucleic acids) that has a stable tertiary structure and is capable of binding to a target molecule with high affinity and specificity.

As long as the aptamer of the present invention and the reaction material interacting with the aptamer generate a detectable signal as a target effect, any kind of aptamer and reaction material may be used.

After hybridization, the promoter probe and reporter probe hybridized to the target nucleic acid sequence are ligated.

In other words, after the promoter probe and the reporter probe of the present invention are hybridized with the target nucleic acid sequence, a nick formed between the two probes is ligated using a ligation agent.

According to a preferred embodiment of the present invention, the promoter probe and the reporter probe are positioned at immediately adjacent locations to each other when hybridized with the target nucleic acid sequence, respectively.

The adjacent positioning is necessary for ligation reactions between the two probes. The term used herein "adjacent" in conjunction with hybridization positions of the promoter probe and the reporter probe means that the 3'-end of one probe and the 5'-end of the other probe are sufficiently close to each other to allow connection of the ends of both probes to one another.

Since enzymatic ligation is the preferred method of linking the promoter probe and the reporter probe by a covalent bond, the term "ligation" is used throughout the application.

Here, the intensity of the fluorescence signal generated may indicate the presence or absence of the target sequence in the reaction product. This assay does not require complicated equipment because all reactions proceed under isothermal (15-50°C) conditions.

Specifically, when the SENSR probe set of the present invention is mixed with the product to be detected (nucleic acid sequence forming a double bond), the temperature is raised to 80~100°C and maintained, and then the temperature is lowered, the SENSR probe set may compete with the remaining complementary sequence of the two strands of the nucleic acid sequence forming a double bond to hybridize with the single-stranded DNA, and if some hybridization is successful, the above SENSR isothermal reaction may occur, resulting in fluorescence.

Here, exposure of the probe hybridization region to the single strand due to the presence of DNA that interferes with double strand recombination helps hybridization between the target DNA and the SENSR probe, further increasing fluorescence generation. In other words, probe hybridization is further triggered.

A more detailed description of the SENSR assay is as follows:
the first probe may be a promoter probe (PP) having a structure of 3'-X-Y-5' (I); wherein X is a stem-loop structure portion containing a promoter sequence recognizable by an RNA polymerase; Y is an upstream hybridization sequence (UHS) portion having a hybridization sequence complementary to a target nucleic acid sequence; the target nucleic acid sequence is DNA or RNA; and X and Y are deoxyribonucleotides; and
the second probe may be a reporter probe (RP) having a structure of 3'-Y'-Z-5' (II); wherein Y' is a downstream hybridization sequence (DHS) portion having a hybridization sequence complementary to the target nucleic acid sequence; Z is an aptamer sequence portion having an interactive labeling system comprising one label or a plurality of labels that generate a detectable signal; the target nucleic acid sequence is DNA or RNA; and Y' and Z are deoxyribonucleotides.

In the present invention, the label may be any one selected from the group consisting of a chemical label, an enzyme label, a radioactive label, a fluorescent label, a luminescent label, a chemiluminescent label, and a metal label.

The isothermal one-pot reaction of the present invention may be performed simultaneously in one vessel, without a separate amplification reaction, unified at any one specified temperature in the range of 15°C to 50°C. The temperature in the range of 15°C to 50°C is a temperature known in the art at which enzymes act, and is not limited thereto, as long as the desired effect of the present invention may be obtained. In an embodiment of the present invention, the specified temperature is preferably 37°C.

The composition for detecting a target nucleic acid according to the present invention includes at least one nucleic acid for interfering with double strand recombination of target gene.

As described above, the purpose of the nucleic acid is to provide, within the nucleic acid sequence forming a double bond, single-stranded nucleic acid (DNA/RNA) fragments (i.e., at least one nucleic acid for interfering with double strand recombination of target gene) that are complementary to the single strand, but are shorter in length than the nucleic acid sequence forming a double bond, thereby achieving a process of forming a single-stranded region of a nucleic acid sequence.

The nucleic acid for interfering with double strand recombination of target gene as described above may have at least 8, 9, 10, 11, 12, 13 or more arbitrary nucleotide sequences, and may have a complementary sequence to the target gene. Approximately 60 or fewer sequences are preferred. Thus, the nucleic acid may have approximately 8 to 60 sequences, including any integer within the above-described numerical range.

This is because the minimum interference effect may be expected only when the above-described nucleic acid for interfering with double strand recombination of target gene has at least 8 arbitrary nucleotide sequences in length. Accordingly, there is no limitation in designing the length of nucleic acid for interfering with double strand recombination of the target gene described above as long as the nucleic acid has any number of nucleotide sequences that are at least 8 in length, but it is desirable to determine the number of nucleotide sequences (length of nucleic acid for interfering with double strand recombination) at a level that does not interfere with the complementary binding of the target gene and the SENSR probe set.

The complementary sequence of the nucleic acid for interfering with double strand recombination may be located at the 3' end or the 5' end of the complementary binding region (i.e., hybridization region) of the SENSR probe set, or both (if there are two or more sequences). More specifically, a sequence complementary to the target nucleic acid sequence that is at least a certain number of nucleotide sequences away is preferred. More specifically, regarding the hybridization region, it is preferable to use a sequence that binds complementary to the target gene at a position approximately 0 to 24, more specifically 5 to 12 nucleotide sequences away from the complementary binding region of the SENSR probe set. The sequence includes any number of sequences within the above range.

In other words, the above-described nucleic acid for interfering double strand recombination serves to inhibit the re-formation of complementary sequences at the 3' end (a sequence of several to tens of nucleotides from the end site) or at the 5' end (a sequence of several to tens of nucleotides from the end site) of the sequence on which the SENSR probe set operates, thereby leaving the region of the sequence open for the SENSR probe set to perform hybridization.

As a result, it is possible to enhance the efficiency of an isothermal reaction.

More specifically, one, two or more nucleic acids for interfering with double strand recombination in the present invention may be used, depending on the location of the mutation to be detected.

When the sequence to be detected is excessively biased toward the 3' or 5' end of the target gene amplicon, in each case, nucleic acids that interfere with double strand recombination at the 3' or 5' end cannot be used because they overlap with the SENSR probe hybridization region. Therefore, in this case, the nucleic acid for interfering with double strand recombination is determined as one complementary sequence at a position (3' or 5' end) opposite to the region (5' or 3' end) where the SENSR probe set operates.

When the sequence to be detected is not biased toward one end of the target gene amplicon, all sequences near the 5' or 3' end of the sequence on which the SENSR probe set operates may be used.

The composition and/or kit of the present invention may also include various polynucleotide molecules, enzymes, various buffers and reagents. Further, the composition and/or kit of the present invention may include reagents essential for performing positive control and negative control reactions. The optimal amount of reagent to be used in any one particular reaction may be easily determined by those skilled in the art having learned the disclosure herein.

The term "ligation" is a general term and is to be understood to include any method of linking two probes by a covalent bond.

The ligation reaction of the present invention may be performed using a wide variety of ligation agents, including enzymatic ligation agents and non-enzymatic ligation agents (e.g., chemical agents and photoligation agents).

Chemical ligation agents include, without limitation, activating, condensing, and reducing agents, such as carbodiimide, cyanogen bromide (BrCN), N-cyanoimidazole, imidazole, 1-methylimidazole/carbodiimide/cystamine, dithiothreitol (DTT) and ultraviolet light.

Autoligation, i.e., spontaneous ligation in the absence of a ligating agent, is also within the scope of the teachings herein.

Photoligation using light of an appropriate wavelength as a ligation agent is also within the scope of the present disclosure. According to certain embodiments, the photoligation comprises probes comprising nucleotide analogs, including but not limited to, 4-thiothymidine (s4T), 5-vinyluracil and its derivatives, or combinations thereof.

According to a preferred embodiment of the present invention, the ligation reaction is performed by an enzymatic ligation agent, wherein the ligation agent includes one selected from the group consisting of SplintR ligase, bacteriophage T4 ligase, *E. coli* ligase, Afu ligase, Tag ligase, Tfl ligase, Mth ligase, Tth ligase, Tth HB8 ligase, *Thermus* species AK16D ligase, Ape ligase, LigTk ligase, Aae ligase, Rm ligase, Pfu ligase, ribozyme and variants thereof.

The internucleotide linkage generated by the ligation includes phosphodiester bond and other linkages. For instance, the ligation using ligases generally produces phosphodiester bonds.

Non-enzymatic methods for ligation may form other internucleotide linkages. Other internucleotide linkages include, without limitation, covalent bond formation between appropriate reactive groups such as a thiophosphorylacetylamino group formed between an α-haloacyl group and a phosphothioate group, a 5'-phosphorothioester formed between a phosphorothioate and tosylate or iodide group, and pyrophosphate linkages.

After the ligation reaction, the resulting ligation product includes an aptamer sequence and becomes single-stranded DNA as a template for amplifying the target RNA/DNA.

Subsequently, when the transcriptional process is initiated by RNA polymerase, the target RNA/DNA is elongated from the single-stranded DNA, which is a template for amplifying the target RNA/DNA containing the aptamer sequence such that signals are generated quickly and simply by the aptamer introduced to fluoresce by binding to specific chemical molecules.

Further, when the RNA aptamer is used as a reporter, the time it takes to observe the generated signal is shortened, compared to a conventional fluorescent signal using a fluorescent protein.

If the promoter probe and the reporter probe are not performed as described above, a signal emitted from the label on the transcriptional product of the promoter probe and the reporter probe is not finally generated, and thus the target nucleic acid sequence is not detected.

The RNA polymerase of the present invention may include any kind of RNA polymerase as long as it can recognize the promoter portion so as to initiate transcription as the desired effect.

Preferably, the RNA polymerase may be selected from the group consisting of bacteriophage T7 RNA polymerase, bacteriophage T3 polymerase, bacteriophage RNA polymerase, bacteriophage ΦII polymerase, *Salmonella* bacteriophage sp6 polymerase, *Pseudomonas* bacteriophage gh-1 polymerase, *E*. *coli* RNA polymerase holoenzyme, *E. coli* RNA polymerase core enzyme, human RNA polymerase I, human RNA polymerase II, human RNA polymerase III, human mitochondrial RNA polymerase and variants thereof, but is not limited thereto.

Likewise, as long as transcription may be initiated by RNA polymerase, any promoter sequence known in the art may be employed for the promoter region in the first probe of the present invention, which is recognized by RNA polymerase.

The ligation agent and the polymerase may be appropriately adjusted to optimize the isothermal one-pot reaction of the present invention. They are included preferably 1 : 1 to 1 : 5 units, more preferably 1 : 4 units, most preferably 1 : 1 units in a one-pot reaction buffer.

The one-pot reaction buffer may be any one selected from the group consisting of Tris-HCl, MgCl₂, NTPs, NaCl, and extreme thermostable single-stranded DNA binding protein (ET-SSB) .

In the present invention, the one-pot reaction buffer preferably includes 1 to 100 mM Tris-HCl; 1 to 50 mM MgCl₂; 0.1 to 10 mM NTPs, and 1 to 50 mM NaCl; and 1 to 800 ng ET-SSB, more preferably, 10 to 60 mM Tris-HCl; 1 to 30 mM MgCl₂; 0.5 to 5 mM NTPs; 1 to 30 mM NaCl; and 100 to 600 ng ET-SSB, most preferably 50 mM Tris-HCl; 10 mM MgCl₂; 1 mM NTPs; 10.5 mM NaCl; and 400 ng ET-SSB.

The type of target is not limited as long as it can be diagnosed by the method of the present invention.

In the present invention, it is preferable to encompass infectious harmful microorganisms, viruses, and/or animals including humans. Here, in the case of animals, the animal's single nucleotide polymorphism (SNP) gene may be used as a detection target.

The target is not limited, but may be, for example, at least one selected from the group consisting of Severe Acute Respiratory Syndrome Coronavirus type 2, *Staphylococcus Aureus, Vibrio vulnificus, Escherichia coli* (*E. coli*), Middle East Respiratory Syndrome Coronavirus, Influenza A virus, Severe Acute Respiratory Syndrome Coronavirus, Respiratory Syndrome Virus (RSV), Human immunodeficiency virus (HIV), herpes simplex virus (HSV), human papilloma virus (HPV), human parainfluenza virus (HPIV), dengue virus, hepatitis B virus (HBV), yellow fever virus, rabies virus, Plasmodium, cytomegalovirus (CMV), mycobacterium tuberculosis, Chlamydia trachomatis, rotavirus, human metapneumovirus (hMPV), Crimean-Congo hemorrhagic fever virus, ebola virus, Zika virus, Henipavirus, Norovirus, Lassa virus, Rhinovirus, Flavivirus, Rift Valley fever virus, Hand, Foot and Mouth disease virus (HFMD), *Salmonella* sp., *Shigella* sp., *Enterobacteriaceae* sp., *Pseudomonas* sp., *Moraxella* sp., *Helicobacter* sp., and *Stenotrophomonas* sp.

Types of SNPs may include rSNP (regulatory SNP), situated in regions related to the regulation of transcriptional activity, such as promoter regions, cSNP (coding SNP) inducing amino acid mutations in exon regions, iSNPs (intronic SNP), exist in intron regions, sSNP (silent SNP) that causes silence mutations in the exon region, and gSNP (genome SNP) that appears in other genomic regions, but are not limited thereto. They may be selected, for example, from SNP genes with known associations with diseases. The method of the present invention is capable of detecting any gene, regardless of its type, provided that its sequence is known.

The composition of the present invention may also be used for detecting two or more target nucleic acid sequences. Here, the composition may be employed to simultaneously detect variants of viruses or harmful bacteria or to detect single nucleotide polymorphism (SNP) mutations by targeting two or more nucleic acid sequences. Accordingly, the present invention provides a composition for detecting a target gene comprising: an isothermal one-pot reaction SENSR first probe set for detecting a target nucleic acid, including a first probe and a second probe; an isothermal one-pot reaction SENSR second probe set for detecting a target nucleic acid mutation, including a third probe and a fourth probe; and at least one nucleic acid for interfering with double strand recombination of target gene. Specifically, the isothermal one-pot reaction probe sets for detecting two or more target nucleic acid sequences each include different interactive labeling systems; each of the two or more probe sets binds to a different target nucleic acid sequence; enabling multiple detection of different target nucleic acid sequences.

In other words, the two or more types of targets may be interactively different molecular diagnostic objects, such as viruses, infectious harmful microorganisms, or animals, including humans. In this case, it is possible to simultaneously detect and diagnose different pathogens. In addition, in this case, it can be used to detect and/or determine the subject's variant or single nucleotide polymorphism (SNP) mutation.

Further, the two or more types of targets may be different target portions present in a single pathogen, and in this case, different portions of the target are effectively detected to enable a more accurate and precise diagnosis.

The composition of the present invention is prepared to perform the detection of a target nucleic acid sequence by the probe set of the present invention as described above. Thus, the overlapping contents are excluded in order to avoid the complexity of the present specification.

For example, in addition to the first and second probes, the third and fourth probes will be described in more detail as follows.

The third probe is a promoter probe (PP) having a structure of 3'-X''-Y' '-5' (I); wherein X'' is a stem-loop structure portion containing a promoter sequence recognizable by an RNA polymerase; Y'' is an upstream hybridization sequence (UHS) portion having a hybridization sequence complementary to the target nucleic acid sequence; the target nucleic acid sequence is DNA or RNA; and X'' and Y'' are deoxyribonucleotides; and

the fourth probe is a reporter probe (RP) having a structure of 3'-Y‴-Z‴-5' (II); Y‴ is a downstream hybridization sequence (DHS) portion having a hybridization sequence complementary to a target SNP nucleic acid sequence; Z‴ is an aptamer sequence portion having an interactive labeling system comprising one label or a plurality of labels that generate a detectable signal; the target nucleic acid sequence is DNA or RNA; and Y‴ and Z‴ are deoxyribonucleotides.

Here, the at least one nucleic acid for interfering with double strand recombination of target gene may bind to the 5' end or 3' end of the single strand of the target gene, excluding the complementary binding regions where each probe set acts.

In other words, these sequences may vary depending on the design of the target sequence.

In other words, when the first probe set and the second probe set use the same promoter sequence, the nucleic acid sequence for interfering with double strand recombination that binds at the 5' end may use the same sequence.

When the detection sites targeted by each probe set are different or target different sequences, the nucleic acid for interfering with double strand recombination may be appropriately applied to each probe set.

In other words, depending on the desired single strand formation, 1, 2, 3, 4 or more nucleic acids for interfering with double strand recombination may be included. More specifically, the above sequence may be a complementary sequence at the 3' end and/or 5' end of the sequence on which each SENSR probe set operates (i.e., hybridization region).

The present invention provides a composition comprising a nucleic acid for interfering with double strand recombination of target gene for an isothermal amplification reaction.

More specifically, the present invention provides a composition for detecting a target nucleic acid comprising a nucleic acid for interfering with double strand recombination of target gene for an isothermal amplification reaction, wherein the nucleic acid for interfering with double strand recombination of target gene is a sequence that binds complementary to the target gene at a position 0 to 24 nucleotide sequences away from a complementary binding region of the probe set.

Target nucleic acids may be detected with high specificity using the nucleic acids for interfering with double strand recombination according to the present invention. As described above, within the nucleic acid sequence forming a double bond, when there are single-stranded nucleic acid (DNA/RNA) fragments that are complementary to the single strand but are shorter in length than the nucleic acid sequence forming a double bond, and the temperature is lowered in the presence of these fragments, the short-length single-stranded nucleic acids (DNAs/RNAs) may be hybridized to interfere with the reformation of the double-stranded structure.

The nucleic acid for interfering with double strand recombination according to the present invention may be located at the 3' end or the 5' end of the complementary binding region (i.e., hybridization region) of the probe set, or both (if there are two or more sequences). More specifically, a sequence complementary to the target nucleic acid sequence that is at least a certain number of nucleotide sequences away is preferred. More specifically, regarding the hybridization region, it is preferable to use a sequence that binds complementary to the target gene at a position approximately 0 to 24, more specifically 5 to 12 nucleotide sequences away from the complementary binding region of the probe set. The sequence includes any number of sequences within the above range.

The nucleic acid for interfering with double strand recombination of target gene as described above may have at least 8, 9, 10, 11, 12, 13 or more arbitrary nucleotide sequences, and may have a complementary sequence to the target gene. Approximately 60 or fewer sequences are preferred. Thus, the nucleic acid may have approximately 8 to 60 sequences, including any integer within the above-described numerical range.

As a result, it is possible to further increase the efficiency of the reaction aimed at isothermal amplification.

In another embodiment of the present invention, the present invention provides a kit comprising: any of the compositions described above; a ligation agent; a polymerase; and an isothermal one-pot reaction buffer.

The kit of the present invention is manufactured to perform the detection of a target nucleic acid sequence by the probe set of the present invention as described above. Thus, the overlapping contents are excluded in order to avoid the complexity of the present specification.

Typically, the kit of the present invention is manufactured in a separate package or compartment including the above-described components.

In still another embodiment of the present invention, the present invention provides a method for detecting a target gene comprising: (a) separating a double strand of the target gene at a high temperature of 80 to 100°C; (b) cooling the reaction product in (a) to an isothermal temperature of 15 to 50°C and then performing an isothermal one-pot reaction by adding the above-described composition; and (c) detecting signal generation.

Here, the detection method may be employed to simultaneously detect variants of viruses or harmful bacteria or to detect single nucleotide polymorphism (SNP) mutations by targeting two or more nucleic acid sequences.

The method according to the present invention has an excellent advantage in that it is possible to exhibit excellent detection efficiency under isothermal one-pot reaction conditions without a separate amplification reaction.

Specifically, the method for detecting a target gene according to the present invention comprises (a) separating the double strand of the target gene at a high temperature of 80 to 100°C. The target gene according to the present invention refers to any nucleic acid sequence forming a double bond. At high temperatures of 80°C or higher, the nucleic acid sequence dehybridizes and represents a single sequence.

The method for detecting a target gene according to the present invention comprises cooling a reaction product in (a) to an isothermal temperature of 15 to 50°C and then performing an isothermal one-pot reaction by adding the composition described above.

As previously described, the addition of the composition described above under isothermal reaction conditions (e.g., approximately 37°C) causes an isothermal reaction to occur. In particular, the presence of the nucleic acid that interferes with double strand recombination may prevent recombination of any nucleic acid sequence forming a double bond as much as possible, thereby allowing the isothermal reaction to occur more smoothly.

The present invention also comprises (c) detecting signal generation.

Here, the detecting of signal generation may indicate the presence of the target gene in the sample.

The detecting of signal generation may be, for example, detection of a signal generated by any one selected from the group consisting of a chemical label, an enzyme label, a radioactive label, a fluorescent label, a luminescent label, a chemiluminescent label, and a metal label.

The composition of the present invention may serve as a powerful diagnostic platform for detecting mutations in DNA or RNA of the target to not only provide a short diagnosis time, high sensitivity and specificity and a simple analysis procedure, but also does not require expensive equipment and diagnostic experts, thereby making it possible to be used as a suitable diagnostic method for detection of SARS-CoV-2 mutations where a rapid response is required.

The present invention also provides a composition for detecting type 2 severe respiratory syndrome virus, comprising: an isothermal one-pot reaction SENSR probe set for type 2 severe respiratory syndrome virus, including a promoter probe of SEQ NO: 1 and a reporter probe of SEQ NO: 2; and a nucleic acid for interfering with double strand recombination of SEQ NO: 6, SEQ NO: 7, or both.

The present invention also provides a composition for detecting type 2 severe respiratory syndrome virus omicron variants, comprising: an isothermal one-pot reaction SENSR probe set for type 2 severe respiratory syndrome virus omicron variants, including a promoter probe of SEQ NO: 8 and a reporter probe of SEQ NO: 9; and a nucleic acid for interfering with double strand recombination of SEQ NO: 10, SEQ NO: 11, or both.

The present invention also provides a composition for simultaneous detection of type 2 severe respiratory syndrome virus and omicron variants thereof, comprising: an isothermal one-pot reaction SENSR probe set for type 2 severe respiratory syndrome virus omicron variants, including a promoter probe of SEQ NO: 8 and reporter probes of SEQ NO: 9 and SEQ NO: 15; and a nucleic acid for interfering with double strand recombination of SEQ NO: 10, SEQ NO: 16, or both. Here, the composition may further comprise: a nucleic acid for interfering with double strand recombination of SEQ NO: 17.

More preferably, the reporter probes of SEQ ID NO: 9 and SEQ ID NO: 15 are designed to be identified by using different aptamers. For example, an aptamer specific to broccoli and an aptamer specific to malachite green may be used in combination.

Specifically, since the spectral range of malachite green is emission: 616 nm, excitation: 665 nm, and the spectral range of broccoli aptamer is emission: 460 nm, excitation: 520 nm, using a composition for detecting two or more SARS-CoV-2 variants together as described above may have the advantage of detecting various mutations at once.

More specifically, the promoter probe comprises a T7 promoter complementary sequence + a loop sequence + a T7 promoter sequence and a hybridization sequence complementary to the target nucleic acid sequence, and the reporter probe comprises an aptamer sequence and a hybridization sequence complementary to the target nucleic acid sequence.

The probe set of the present invention may serve as a powerful diagnostic platform for detecting RNA of the target to not only provide a short diagnosis time, high sensitivity and specificity and a simple analysis procedure, but also does not require expensive equipment and diagnostic experts, thereby making it possible to be used as a suitable diagnostic method for detection of SARS-CoV-2 mutations where a rapid response is required.

The present invention also provides a kit for detecting type 2 severe respiratory syndrome virus or omicron variants thereof, comprising: these composition described above; a ligation agent; a polymerase; and an isothermal one-pot reaction buffer.

In particular, when using the composition for simultaneous detection of type 2 severe respiratory syndrome virus and omicron variants thereof, each probe set comprises different interactive labeling systems; and each binds to a different target nucleic acid sequence; which allows multiple detection of different target nucleic acid sequences. In other words, it is possible to perform simultaneous detection and diagnosis of type 2 severe respiratory syndrome virus and omicron variants thereof.

The kit of the present invention is manufactured to perform the detection of a target nucleic acid sequence by the probe set of the present invention as described above. Thus, the overlapping contents are excluded in order to avoid the complexity of the present specification.

The kit of the present invention as described above may additionally include various polynucleotide molecules, enzymes, various buffers and reagents. Further, the kit of the present invention may include reagents essential for performing positive control and negative control reactions. The optimal amount of reagent to be used in any one particular reaction may be easily determined by those skilled in the art having learned the disclosure herein.

Typically, the kit of the present invention is manufactured in a separate package or compartment including the above-described components.

The kit of the present invention is manufactured to perform the detection of a target nucleic acid sequence by the probe set of the present invention as described above. Thus, the overlapping contents are excluded in order to avoid the complexity of the present specification.

The method of the present invention comprises the probe set of the present invention described above, and thus overlapping contents are excluded in order to avoid excessive complexity of the present specification.

Further, the present invention provides a diagnostic method for detecting target SARS-CoV-2 or omicron variants thereof, comprising: (a) separating a double strand of a target gene at a high temperature of 80 to 100°C; (b) cooling a reaction product in (a) to an isothermal temperature of 15 to 50°C and then performing an isothermal one-pot reaction by adding the composition described above; and (c) confirming luminescence.

As described above, when the isothermal one-pot reaction probe set is processed on the same sample, it is possible to perform detection in one pot by varying the fluorescence capable of detecting the signal generation of the aptamer.

The isothermal one-pot reaction of the present invention, without a separate amplification reaction, is performed simultaneously while being unified at any one of the specified temperatures in the range of 15°C to 50°C, and preferably, the specified temperature is 37°C.

The isothermal one-pot reaction is performed simultaneously while being unified with a one-pot reaction buffer containing Tris-HCl, MgCl₂, NTPs, NaCl, and extreme thermostable single-stranded DNA binding protein (ET-SSB).

In addition, the present invention is characterized in that an additional separate amplification process (e.g., PCR) is not required, and the amplification process is performed automatically during an isothermal one-pot reaction.

The feasibility of the isothermal one-pot reaction including an amplification process is achieved because of (i) the design of the reporter probe to form an aptamer downstream due to a ligation reaction occurring with the promoter probe including the double-stranded promoter sequence having a hairpin structure; and (ii) the amplification process that occurs naturally in the reaction without a separate amplification process, by using what may be used as a target sequence (i.e., splint) when the ligated product is transcribed by transcription initiation.

That is, when the ligation product of the ligation reaction between the promoter probe and the reporter probe is subjected to transcription initiation from the RNA polymerase promoter sequence of the promoter probe that forms a hairpin structure and a transcript is then produced, the transcript contains the same sequence as the target RNA sequence to thereby be usable as the target RNA. In addition, since the transcribed ligation product includes the same sequence as the target nucleic acid sequence, it may act as a target RNA among target nucleic acids.

Therefore, the platform using the probe set of the present invention is designed to allow ligation, transcription reaction, and fluorescence reaction to occur simultaneously, which is an isothermal one-pot reaction in which the transcript formed by the transcription reaction from the ligated product is used as a splint RNA, thereby enabling the amplification process to be included.

Accordingly, steps (a) to (c) of the present invention may be performed simultaneously in one vessel, such as a tube.

In addition, the efficiency of the above isothermal one-pot reaction may be further increased by additionally providing the nucleic acid for interfering with double strand recombination of target gene of the present invention.

In the present invention, the sequences of the DNA probe are not limited to the sequences described in the Examples of the present invention as long as the desired effect is achieved, but are applicable to all target gene sequences. In addition, the aptamer used for final signal confirmation is not limited to the malachite green aptamer, and may be any type of RNA-based fluorescent aptamer.

Further, the present disclosure provides a method for providing information for single nucleotide polymorphism (SNP)-based disease diagnosis, comprising: (a) separating a double strand of a target gene at a high temperature of 80 to 100°C; (b) cooling a reaction product in (a) to an isothermal temperature of 15 to 50°C and then performing an isothermal one-pot reaction by adding the composition according to claim 8; (c) detecting signal generation; and (d) determining the onset of a genetic disease when a mutation at the level of single nucleotide polymorphism (SNP) in the gene is confirmed.

According to an embodiment of the present invention, experiments conducted on representatively known SNP genes showed that mutations could be specifically detected in all SNP genes.

Accordingly, as disease diagnosis methods targeting SNPs are being actively studied, diagnosis of genetic diseases related to SNP mutations will be significantly facilitated by employing the method of the present invention for mutations in SNPs known to be associated with diseases.

As examples, SNP mutations in the LOC102724084, MINK1, TCF24, RNF144A, and ARHGAP32 genes are known to be associated with the development of cerebral aneurysms (KR 10-2158721 B1, etc.), and rs1924089, rs1039239, rs11064191, and the like, among the known SNPs, are known to be associated with the development of chronic sensorineural tinnitus (KR 10-2022-0025985 A1).

### [Advantageous Effects]

The detection method according to the present invention is able to detect sequence-specific nucleic acids and detect mutations at the single nucleotide polymorphism (SNP) discrimination level, thereby allowing for the accurate and sensitive discrimination of mutations in a short time, and thus, it is possible to provide an effective mutation detection method capable of responding not only to current SARS-CoV-2 variants but also generally to specific diseases or related mutations in the future.

### [Description of Drawings]

FIG. 1 is a schematic diagram illustrating comparison of the actions of a probe set including double strand recombination-interfering DNA (left) according to the present invention and a SENSR probe set (right).
FIG. 2 is a schematic diagram showing the action of the SENSR probe set.
FIG. 3 shows the detection results of PCR amplicon using the SENSR probe set.
FIG. 4 is a schematic diagram of the action of a probe set including the SENSR probe and a downstream double strand recombination-interfering DNA probe.
FIG. 5 shows the detection results of PCR amplicon using the probe set including the SENSR probe and the downstream double strand recombination-interfering DNA probe.
FIG. 6 is a schematic diagram showing the action of a probe set including the SENSR probe and upstream and downstream double strand recombination-interfering DNA probes.
FIG. 7 shows the detection results of PCR amplicon using the probe set including the SENSR probe and the downstream double strand recombination-interfering DNA probe; and the probe set including the SENSR probe and the upstream and downstream double strand recombination-interfering DNA probes.
FIG. 8 shows the comparison results of the detection specificity between SARS-CoV-2 and omicron variant thereof using a probe set including upstream and downstream double strand recombination-interfering DNA probes.
FIG. 9 shows the comparison results of the detection specificity of SNP mutations of MT-CO2(COX2), mitochondrial housekeeping gene, using the probe set including the downstream double strand recombination-interfering DNA probe.
FIG. 10 shows the comparison results of the detection specificity of SNP mutations of TP53, tumor suppressor gene, using the probe set including the downstream double strand recombination-interfering DNA probe.
FIG. 11 shows the comparison results of the detection specificity of SNP mutations of ACTB, β-actin gene, using the probe set including the downstream double strand recombination-interfering DNA probe.

### [Best Mode]

Hereinafter, the present invention will be described in more detail through Examples. These Examples are provided solely for the purpose of illustrating the present invention, and it will be apparent to those skilled in the art that the scope of the present invention should not be construed as limited by these Examples.

### Experimental Materials

T7 RNA polymerase to synthesize RNA used in the present experiment was purchased from New England Biolabs (NEB, Ipswich, MA, USA), and dithiothreitol (DTT) was purchased from Thermo Fisher Scientific (Waltham, MA, USA). In addition, Tris-HCl (pH 7.4) and 1M MgCl₂, which are reaction buffer materials required for this reaction, were purchased from Bioneer, Inc (Daejeon, Republic of Korea). In addition, SplintR ligase, ET-SSB (Extreme Thermostable Single-stranded DNA binding protein), DNase I, and ribonucleotide solution mix were purchased from New England Biolabs (NEB, Ipswich, MA, USA).

Further, ATP was purchased from Thermo Fisher Scientific (Waltham, MA, USA), and Recombinant RNase Inhibitor (RRI) was purchased from Takara (Kyoto, Japan). Malachite green oxalate, a fluorescent dye, was purchased from Sigma-Aldrich (St. Louis, MO, USA), and all single-stranded DNAs used as probes were custom-made through Bioneer.

In addition, oligonucleotides for template DNA and double strand recombination-interfering DNAs required to synthesize RNA were purchased from Cosmogenetech, Inc. (Seoul, Republic of Korea) for synthesis. TOPreal qPCR 2X PreMIX (SYBR Green with high ROX) for real-time PCR amplicons was purchased from Enzynomics, Inc. (Daejeon, Republic of Korea), and the GCDia^{™} COVID-19 Fast Detection Kit for performing the rRT-PCR process was provided by Genes Laboratories (Seongnam, Republic of Korea). Kits for PCR purification and RNA purification were all purchased from GeneAll Biotechonology (Seoul, Republic of Korea).

### Example 1. Detection of SARS-CoV-2 using SENSR probe set

Previously developed RNA detection assay [Sensitive splint-based one-pot isothermal RNA detection (SENSR); hereinafter, referred to as SENSR assay; Nat Biomed Eng 4, 1168-1179 (2020)] was used to design the corresponding probe set, and the information is shown in Table 1 below.

**[Table 1]**

| **Sequence type** | **Sequence information (5' → 3')** |
|---|---|
| **Promoter probe (SEQ ID NO: 1)** | [phosphate]TTCAAGGCTCCCTCAGTTGCCCCTAT AGTGAGTCGTATTAATTTCGCGACAACACGCGAAATT AATACGACTCACTATAGGG |
| **Reporter probe (SEQ ID NO: 2)** | GGATCCATTCGTTACCTGGCTCTCGCCAGTCGGGATC CGTGCCAATGTGATCTTTTGGTGTA |
| **SARS-CoV-2 target sequence (SEQ ID NO: 3)** | GACGGCATCATATGGGTTGCAACTGAGGGAGCCTTGA ATACACCAAAAGATCACATTGGCACCCGCAATCCTGC TAACAATGCTGCAATCGTGCTACAACTTCCTCAAGGA ACAACATTGCCAAAAGGCTTCTACGCAGAAGGGAGCA GAGGCGGCAGTCAAGCCTCTTCTCGTTCCTCATCACG TAGTCGCAACAGTTCAAGAAATTCAACTCCAGGCAGC |
| **SARS-CoV-2 PCR primer 1 (SEQ ID NO: 4)** | GACGGCATCATATGGGTTG |
| **SARS-CoV-2 PCR primer 2 (SEQ ID NO: 5)** | GCTGCCTGGAGTTGAATTTCTT |

Meanwhile, the process of performing the SENSR reaction for SARS-CoV-2 detection is shown below. Specifically, the PCR reactant in Table 2 was reacted at 98°C for 5 minutes to denature the double-stranded DNA. Then, the reactant was cooled to 37°C, and the SENSR reactant in Table 3 was added and reacted at 37°C for 15 minutes. Further, in order to measure the fluorescence resulting from the above reaction, 20 µl of the reaction product was dispensed into a 384-well clear flat-bottom black microplate. Then, the microplate was placed in a microplate reader (Hidex Sense), and the fluorescence was measured after setting the absorption/emission wavelength for the fluorescent dye (absorption: 616 nm / emission: 665 nm for malachite green).

**[Table 2]**

| PCR reactant | | |
|---|---|---|
| **Reagent** | **Concentration** | **Amount** |
| **Purified PCR resultant** | 1 µM | 6 **µℓ** |
| **Promoter probe (PP)** | 10 µM | 0.6 **µℓ** |
| **Reporter probe (RP)** | 10 µM | 1.32 **µℓ** |
| **SENSR buffer** | 10X | 1.08 **µℓ** |
| **RNase-free water** | | 11 **µℓ** |
| **Total** | | 20 **µℓ** |

**[Table 3]**

| SENSR Reactant | | |
|---|---|---|
| **Reagent** | **Concentration** | **Amount** |
| **Sample (Resultant i)** | 300 nM | 9 **µℓ** |
| **SENSR buffer** | 10X | 3 **µℓ** |
| **NTPs** | 25 mM each | 3 **µℓ** |
| **ATP** | 1 M | 0.3 **µℓ** |
| **ET-SSB** | 500 ng/**µℓ** | 1 **µℓ** |
| **RNase Inhibitor** | 40 U/**µℓ** | 1 **µℓ** |
| **SplintR ligase** | 25 U/**µℓ** | 3 **µℓ** |
| **T7 RNA polymerase** | 50 U/**µℓ** | 1.5 **µℓ** |
| **Malachite green** | 320 µM | 1.5 **µℓ** |
| **RNase-free water** | | Up to 30 µℓ |
| **Total** | | 30 **µℓ** |

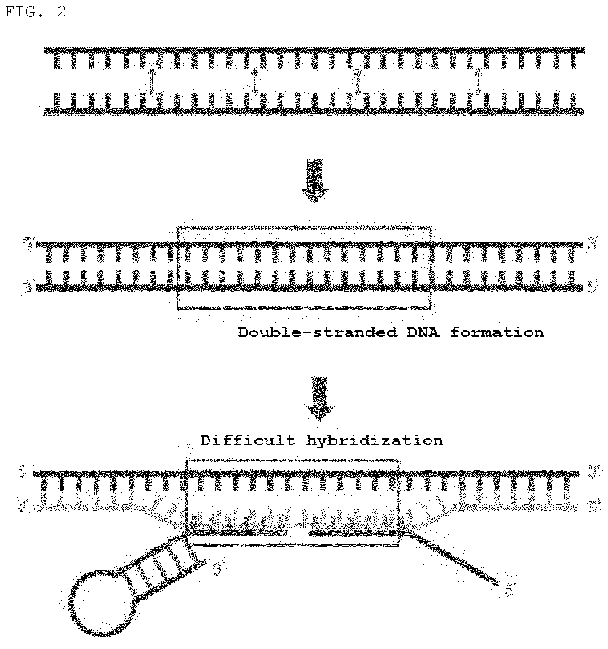

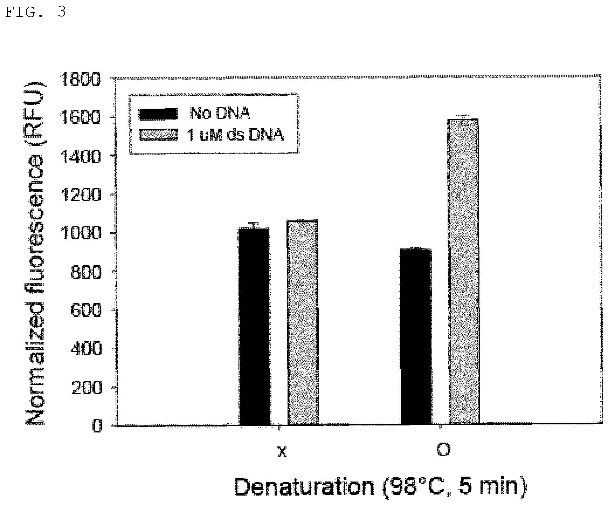

As a result of the experiment, as shown in FIG. 3, specific detection was possible when SARS-CoV-2 was present. As a result of separating the double strand of SARS-CoV-2 by inducing a denaturation process at 98°C for 5 minutes, it was confirmed that the binding of the SENSR probe was well achieved during the cooling process. A schematic diagram of this reaction is shown in FIG. 2.

### Example 2. Improvement of detection efficiency using double strand recombination-interfering nucleic acid (downstream)

In order to improve the hybridization degree of the probe confirmed in Example 1, double strand recombination-interfering DNA located at the downstream (posterior) of the hybridization region was introduced. Double strand recombination-interfering DNA is a short-length single-stranded DNA capable of hybridizing with the single strand of the PCR amplicon and capable of hybridizing to the remaining PCR amplification regions except the SENSR hybridization region.

Double strand recombination-interfering DNA located at the downstream of the hybridization region to increase the detection efficiency for SARS-CoV-2 was constructed, and the information is shown in Table 4 below. Sequences designed to bind to the 3' end of the sense sequence and each having a length of 30 or 40 nt were used.

In addition, the experiment was performed in the same manner as in Example 1.

**[Table 4]**

| **Sequence type** | **Sequence information (5' → 3')** |
|---|---|
| **Double strand recombinatio n-interfering DNA (30 nt) (SEQ ID NO: 6)** | CAATGTTGTTCCTTGAGGAAGTTGTAGCAC |
| **Double strand recombinatio n-interfering DNA (40 nt) (SEQ ID NO: 7)** | CAATGTTGTTCCTTGAGGAAGTTGTAGCACGATTGCAGCA |

**[Table 5]**

| PCR reactant | | |
|---|---|---|
| **Reagent** | **Concentration** | **Amount** |
| **Purified PCR resultant** | 1 µM | 6 **µℓ** |
| **Intruder DNA** | 1 µM | 6 **µℓ** |
| **Promoter probe (PP)** | 10 µM | 0.6 **µℓ** |
| **Reporter probe (RP)** | 10 µM | 1.32 **µℓ** |
| **SENSR buffer** | 10X | 1.08 **µℓ** |
| **RNase-free water** | | 5 **µℓ** |
| **Total** | | 20 **µℓ** |

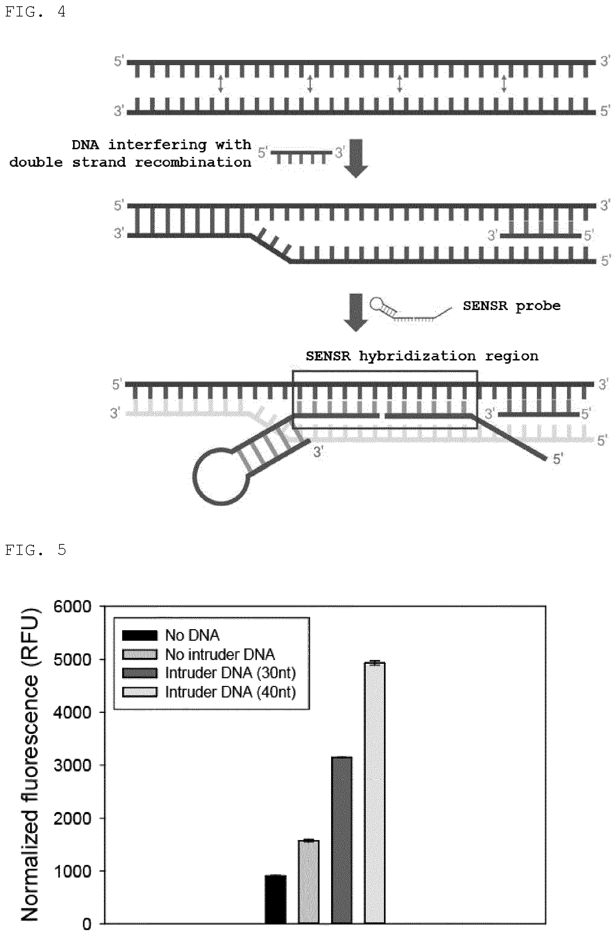

As a result of the experiment, as shown in FIG. 5, it was confirmed that the introduction of double strand recombination-interfering DNA increased the fluorescence intensity according to the reaction compared to the absence of double strand recombination-interfering DNA, thereby further improving the hybridization degree of the probe. A schematic diagram of this reaction is shown in FIG. 4.

### Example 3. Improvement of detection efficiency using double strand recombination-interfering nucleic acids (upstream and downstream)

In Example 2, it was confirmed that target detection efficiency was improved due to the presence of the double strand recombination-interfering nucleic acid located at the downstream (posterior) of the hybridization region, and in order to further improve the hybridization degree of the identified probe, double strand recombination-interfering DNA located at the upstream (anterior) of the hybridization region was additionally introduced.

In Example 3, an attempt was made to detect omicron variants, and the corresponding probe set and double strand recombination-interfering DNAs located at the downstream (posterior) and the upstream (anterior) of the hybridization region were constructed. The information thereof is shown in Table 6 below. Further, the experiment was conducted in the same manner as Example 2.

**[Table 6]**

| **Sequence type** | **Sequence information (5' → 3')** |
|---|---|
| **Promoter probe (SEQ ID NO: 8)** | [Phosphate]GTGCATTTCGCTGATTTTGGGGCCCTA TAGTGAGTCGTATTAATTTCGCGACAACACGCGAAATT AATACGACTCACTATAGGG |
| **Reporter probe (SEQ ID NO: 9)** | GGATCCATTCGTTACCTGGCTCTCGCCAGTCGGGATCC GTCCACCAAACGTAATGCGGA |
| **Downstream double strand recombination-interfering DNA (SEQ ID NO: 10)** | GGGGCCGACGTTGTTTTGATCGCGCCCCACCATTCTGG TTACTGCCAGTT |
| **Upstream double strand recombination-interfering DNA (SEQ ID NO: 11)** | TATCAGACAT |
| **Omicron target sequence (SEQ ID NO: 12)** | ATGTCTGATAATGGACCCCAAAATCAGCGAAATGCACT CCGCATTACGTTTGGTGGACCCTCAGATTCAACTGGCA GTAACCAGAATGGTGGGGCGCGATCAAAACAACGTCGG CCCCAAGGTTTACCCA |
| **Omicron PCR primer 1 (SEQ ID NO: 13)** | GACCCCAAAATCAGCGAAAT |
| **Omicron PCR primer 2 (SEQ ID NO: 14)** | TGGGTAAACCTTGGGGC |

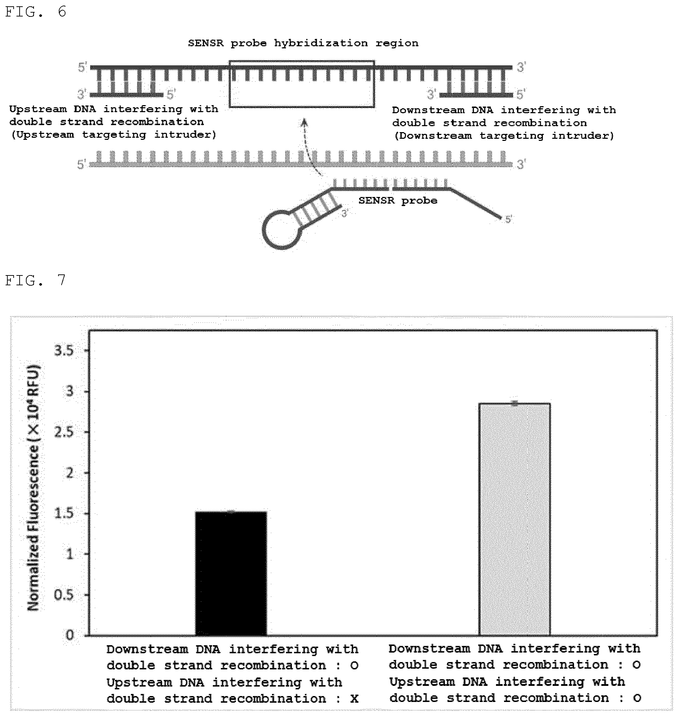

As a result of the experiment, as shown in FIG. 7, it was confirmed that the introduction of the additional short double strand recombination-interfering DNA at the upstream of the hybridization region increased the fluorescence intensity, thereby further improving the hybridization degree of the probe. A schematic diagram of this reaction is shown in FIG. 6.

### Example 4. Discrimination of variants using rRT-PCR resultants

By applying the sequence-specific DNA detection method using the PCR amplicon identified in Experimental Example 3, an attempt was made to distinguish SARS-CoV-2 and variant thereof, the omicron variant.

The rRT-PCR probe sequences, double strand recombination-interfering DNA sequences (upstream, downstream), and SENSR probe sequences for detecting SAR-CoV-2 and omicron variants used in this method are shown in Table 7 below.

**[Table 7]**

| **Sequence type** | **Sequence information (5' → 3')** |
|---|---|
| **Promoter probe (SEQ ID NO: 8)** | [Phosphate]GTGCATTTCGCTGATTTTGGGGCCCTAT AGTGAGTCGTATTAATTTCGCGACAACACGCGAAATTAA TACGACTCACTATAGGG |
| **SARS-CoV-2 target Reporter probe (SEQ ID NO: 15)** | GGATCCATTCGTTACCTGGCTCTCGCCAGTCGGGATCCT CCACCAAACGTAATGCGGG |
| **Omicron target Reporter probe (SEQ ID NO: 9)** | GGATCCATTCGTTACCTGGCTCTCGCCAGTCGGGATCCG TCCACCAAACGTAATGCGGA |
| **Downstream double strand recombination-interfering DNA 1 (SEQ ID NO: 16)** | TTGGGGCCGACGTTGTTTTGATCGCGCCCCACTGCGTTC TCCATTCTGGTT ACTGCCAG |
| **Downstream double strand recombination-interfering DNA 2 (SEQ ID NO: 10)** | GGGGCCGACGTTGTTTTGATCGCGCCCCACCATTCTGGT TACTGCCAGTT |
| **Upstream double strand recombination-interfering DNA (SEQ ID NO: 17)** | TATCAGACAT |
| **SARS-CoV-2 target sequence (SEQ ID NO: 18)** | ATGTCTGATAATGGACCCCAAAATCAGCGAAATGCACCC CGCATTACGTTTGGTGGACCCTCAGATTCAACTGGCAGT AACCAGAATGGAGAACGCAGTGGGGCGCGATCAAAACAA CGTCGGCCCCAAG |
| **Omicron target sequence (SEQ ID NO: 12)** | ATGTCTGATAATGGACCCCAAAATCAGCGAAATGCACTC CGCATTACGTTTGGTGGACCCTCAGATTCAACTGGCAGT AACCAGAATGGTGGGGCGCGATCAAAACAACGTCGGCCC CAAGGTTTACCCA |
| **SARS-CoV-2 PCR primer 1 (SEQ ID NO: 19)** | GACCCCAAAATCAGCGAAAT |
| **SARS-CoV-2 PCR primer 2 (SEQ ID NO: 20)** | TCTGGTTACTGCCAGTTGAATCTG |
| **Omicron PCR primer 1 (SEQ ID NO: 13)** | GACCCCAAAATCAGCGAAAT |
| **Omicron PCR primer 2 (SEQ ID NO: 14)** | TGGGTAAACCTTGGGGC |

Meanwhile, the rRT-PCR process was performed to convert the transcribed RNA into cDNA, followed by amplification to obtain a PCR product. The components required for the reaction are shown in Table 8 below. Further, the reaction product of rRT-PCR was reacted under the condition shown in Table 9 below to obtain a reaction product. Then, the rRT-PCR reaction product was mixed with double strand recombination-interfering DNA and the SENSR probe set, and the reaction was performed under the conditions in Table 10. Further, the resulting reaction product was reacted at 98°C for 5 minutes to denature double-stranded DNA. Then, the reaction product was cooled to 37°C, and the SENSR reactant in Table 11 was added and reacted at 37°C for 15 minutes. Further, in order to measure the fluorescence resulting from the above reaction, 20 µl of the reaction product was dispensed into a 384-well clear flat-bottom black microplate. Then, the microplate was placed in a microplate reader (Hidex Sense), and the fluorescence was measured after setting the absorption/emission wavelength for the fluorescent dye (absorption: 616 nm / emission: 665 nm for malachite green).

**[Table 8]**

| **Reagent** | **Concentration** | **Amount** |
|---|---|---|
| **Primer** | | 10 pmol |
| **Template RNA** | Variable | |
| **GCdia**^{™} **Fast Master mixture** | 2 X | 10 **µℓ** |
| **Nuclease-free water** | | Up to 20 **µℓ** |
| **Total** | | 20 **µℓ** |

**[Table 9]**

| **PCR step** | **Tm** | **Duration** | **Cycle** |
|---|---|---|---|
| **Reverse Transcription** | 50°C | 5 min | 1 |
| **Pre-Heating** | 95°C | 30 sec | 1 |
| **Pre-amplification** | 95°C | 1 sec | 5 |
| | 64°C | 10 sec | |
| **Denaturation** | 95°C | 1 sec | 35 |
| **amplification** | 64°C (Melting temperature) | 10 sec | |

**[Table 10]**

| **Reagent** | **Concentration** | **Amount** |
|---|---|---|
| **rRT-PCR resultant** | Variable | 6 **µℓ** |
| **Downstream double strand recombination-interfering DNA** | 20 mM | 3 **µℓ** |
| **Upstream double strand recombination-interfering DNA** | 20 mM | 3 **µℓ** |
| **Promoter probe (PP)** | 10 µM | 0.6 **µℓ** |
| **Reporter probe (RP)** | 10 µM | 1.32 **µℓ** |
| **SENSR buffer** | 10X | 1.08 **µℓ** |
| **RNase-free water** | | 3 **µℓ** |
| **Total** | | 18 **µℓ** |

**[Table 11]**

| **Reagent** | **Concentration** | **Amount** |
|---|---|---|
| **Sample (Resultant i)** | - | 9 **µℓ** |
| **SENSR buffer** | 10X | 3 **µℓ** |
| **NTPs** | 25 mM each | 3 **µℓ** |
| **ATP** | 1 M | 0.3 **µℓ** |
| **ET-SSB** | 500 ng/**Mℓ** | 1 **µℓ** |
| **RNase Inhibitor** | 40 U/**Mℓ** | 1 **µℓ** |
| **SplintR ligase** | 25 U/**Mℓ** | 3 **µℓ** |
| **T7 RNA polymerase** | 50 U/**Mℓ** | 1.5 **µℓ** |
| **Malachite green** | 320 **µ**M | 1.5 **µℓ** |
| **RNase-free water** | | Up to 30 **µℓ** |
| **Total** | | 30 **µℓ** |

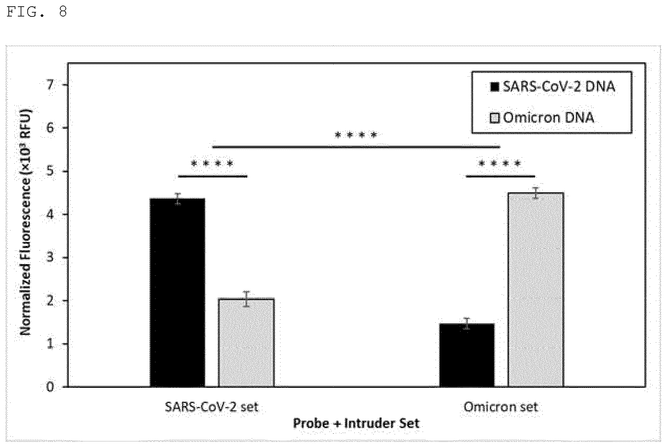

As a result of the experiment, as shown in FIG. 8, it was confirmed that SARS-CoV-2 and Omicron variants could be easily discriminated and detected from PCR amplicon by the reaction of about 20 minutes. Specifically, it could be confirmed that the difference in fluorescence values depending on the mutation of the target gene in the two probes for detecting SARS-CoV-2 and omicron variant is statistically significant (t-test, ****: p<0.0001), and each probe could specifically distinguish the presence or absence of mutation (two-way ANOVA test, ****: p<0.0001). This result implies that it is possible to distinguish the presence or absence of mutation in virus by using the difference in signal intensity between the two viruses.

### Example 5. Human genome SNP discrimination and detection

Up to the previous Examples, the sequence-specific DNA detection method using PCR amplicon was applied to detect various types of viral targets. However, the present method may be used not only for viruses but also for other targets. As an example, an attempt was made to discriminate and detect representative SNPs in the human genome.

For this purpose, three human genome SNPs were used as targets: MT-CO2 (COX2) (mitochondrial housekeeping gene), TP53 (tumor suppressor gene), and ACTB (β-actin gene).

The SNP location and the base to be altered in each gene, and the names of the probes used in the present reaction are shown in Table 12 below.

**[Table 12]**

| **Gene Name** | **Location / Base** | **Probe Name** |
|---|---|---|
| **MT-CO2 (COX2)** | 186 / A | Gene name_target |
| | 186 / C | Gene name_non-target |
| **TP53** | 524 / G | Gene name_target |
| | 524 / A | Gene name_non-target |
| **ACTB** | 776 / A | Gene name_target |
| | 776 / C | Gene name_non-target |

The probe sequences, the double strand recombination interfering DNA sequences (upstream, downstream), and the primer sequences for synthesizing each target used in the present method are shown in Tables 13 to 15 below.

**[Table 13]**

| **Gene: MT-CO2 (COX2)** | |
|---|---|
| **Sequence Type** | **Sequence Information (5' → 3')** |
| **COX2_target promoter probe (SEQ ID NO: 21)** | [Phosphate]TTCTATTTCCTGAGCGTCTGAG ATCCCTATAGTGAGTCGTATTAATTTCGCGACA ACACGCGAAATTAATACGACTCACTATAGGG |
| **COX2_target reporter probe (SEQ ID NO: 22)** | GTATGTGGGAGCCCACACTCTACTCGACAGATA CGAATATCTGGACCCGACCGTCTCCCACATACG GCAGGATAGTTCAGACGGT |
| **COX2_non-target promoter probe (SEQ ID NO: 23)** | [Phosphate]CTCTATTTCCTGAGCGTCTGAG ATCCCTATAGTGAGTCGTATTAATTTCGCGACA ACACGCGAAATTAATACGACTCACTATAGGG |
| **COX2_non-target reporter probe** | GTATGTGGAGCCCACACTCTACTCGACAGATA CGAATATCTGGACCCGACCGTCTCCCACATACG |
| **(SEQ ID NO: 24)** | GCAGGATAGTTCAGACGGT |
| **Downstream double strand recombination-interfering DNA1, COX2 (SEQ ID NO: 25)** | TTATGTAAAGGATGCGTAGGGATGGGAGGGCGA TGAGGACTAGGATGATGGCG |
| **Downstream double strand recombination-interfering DNA 2, COX2 (SEQ ID NO: 26)** | TTATGTAAAGGATGCGTAGGGATGGGAGGGCGA TGAGGACTAGGATGA |
| **Upstream double strand recombination-interfering DNA, COX2 (SEQ ID NO: 27)** | GTTAGTATTAGTTAGTTTTGTTGTGAGTGTTAG GAAAAGGGCATACAGGACTA |
| **COX2_target sequence (SEQ ID NO: 28)** | TAGTCCTGTATGCCCTTTTCCTAACACTCACAA CAAAACTAACTAATACTAACATCTCAGACGCTC AGGAAATAGAAACCGTCTGAACTATCCTGCCCG CCATCATCCTAGTCCTCATCGCCCTCCCATCCC TACGCATCCTTTACATAA |
| **COX2_non-target sequence (SEQ ID NO: 29)** | TAGTCCTGTATGCCCTTTTCCTAACACTCACAA CAAAACTAACTAATACTAACATCTCAGACGCTC AGGAAATAGAGACCGTCTGAACTATCCTGCCCG CCATCATCCTAGTCCTCATCGCCCTCCCATCCC TACGCATCCTTTACATAA |
| **COX2_target PCR primer 1 (SEQ ID NO: 30)** | TAGTCCTGTATGCCCTTTTCCTAACACTCACAA CAAAACTAACTAATACTAACATCTCAGACGCTC AGGAAATAGAAACCGTC |
| **COX2_target PCR primer 2 (SEQ ID NO: 31)** | TTATGTAAAGGATGCGTAGGGATGGGAGGGCGA TGAGGACTAGGATGATGGCGGGCAGGATAGTTC AGACGGTTTCTATTTCCTGAGCG |
| **COX2_non-target PCR primer 1 (SEQ ID NO: 32)** | TAGTCCTGTATGCCCTTTTCCTAACACTCACAA CAAAACTAACTAATACTAACATCTCAGACGCTC AGGAAATAGAGACCGTCTGAAC |
| **COX2_non-target PCR primer 2 (SEQ ID NO: 33)** | TTATGTAAAGGATGCGTAGGGATGGGAGGGCGA TGAGGACTAGGATGATGGCGGGCAGGATAGTTC AGACGGTCTCTATTTCCTGAG |
| **COX2_PCR primer 1 (SEQ ID NO: 34)** | TACTCCTGTATGCCCTTTTCCTAACA |
| **COX2_PCR primer 2 (SEQ ID NO: 35)** | TATGTAAAGGATGCGTAGGGATGG |

**[Table 14]**

| **Gene: TP53** | |
|---|---|
| **Sequence Type** | **Sequence Information (5' → 3')** |
| **TP53_target promoter probe (SEQ ID NO: 36)** | [Phosphate]ATCCCACACCCTGGAGG CCCTATAGTGAGTCGTATTAATTTCGCGACAAC ACGCGAAATTAATACGACTCACTATAGGG |
| **TP53_target reporter probe** | GTATGTGGGAGCCCACACTCTACTCGACAGATA |
| **(SEQ ID NO: 37)** | CGAATATCTGGACCCGACCGTCTCCCACATACG TACCTAAAAGGAAATCTCACCCC |
| **TP53_non-target promoter probe (SEQ ID NO: 38)** | [Phosphate]ATCCCACACCCTGGAGGCCCTA TAGTGAGTCGTATTAATTTCGCGACAACACGCG AAATTAATACGACTCACTATAGGG |
| **TP53_non-target reporter probe (SEQ ID NO: 39)** | GTATGTGGGAGCCCACACTCTACTCGACAGATA CGAATATCTGGACCCGACCGTCTCCCACATACG TACCTAAAAGGAAATCTCACCCT |
| **Downstream double strand recombination-interfering DNA 1, TP53 (SEQ ID NO: 40)** | TTGTCAAGTCTCTGCTGGCCCAGCCAAACCCTG TCTGACAACCTCTTGGTGAACCTTA |
| **Downstream double strand recombination-interfering DNA 2, TP53 (SEQ ID NO: 41)** | TTGTCAAGTCTCTGCTGGCCCAGCCAAACCCTG TCTGACAACC |
| **Upstream double strand recombination-interfering DNA1, TP53 (SEQ ID NO: 42)** | TTGTATATGATGATCTGGATCCACCAAGACTTG TTTTATGC |
| **Upstream double strand recombination-interfering DNA2, TP53 (SEQ ID NO: 43)** | ATCTCTTGTATATGATGATCTGGATCCACCAAG ACTTGTTTTATGC |
| **TP53_target sequence (SEQ ID NO: 44)** | GCATAAAACAAGTCTTGGTGGATCCAGATCATC ATATACAAGAGATGAAATCCTCCAGGGTGTGGG ATGGGGTGAGATTTCCTTTTAGGTACTAAGGTT CACCAAGAGGTTGTCAGACAGGGTTTGGCTGGG CCAGCAGAGACTTGACAA |
| **TP53_non-target sequence (SEQ ID NO: 45)** | GCATAAAACAAGTCTTGGTGGATCCAGATCATC ATATACAAGAGATGAAATCCTCCAGGGTGTGGG ATAGGGTGAGATTTCCTTTTAGGTACTAAGGTT CACCAAGAGGTTGTCAGACAGGGTTTGGCTGGG CCAGCAGAGACTTGACAA |
| **TP53_target PCR primer 1 (SEQ ID NO: 46)** | GCATAAAACAAGTCTTGGTGGATCCAGATCATC ATATACAAGAGATGAAATCCTCCAGGGTGTGGG ATGGGGTGAGATTTC |
| **TP53_target PCR primer 2 (SEQ ID NO: 47)** | TTGTCAAGTCTCTGCTGGCCCAGCCAAACCCTG TCTGACAACCTCTTGGTGAACCTTAGTACCTAA AAGGAAATCTCACCCCATCCCAC |
| **TP53_non-target PCR primer 1 (SEQ ID NO: 48)** | GCATAAAACAAGTCTTGGTGGATCCAGATCATC ATATACAAGAGATGAAATCCTCCAGGGTGTGGG ATAGGGTGAGATTTCCTTTTAG |
| **TP53_non-target PCR primer 2 (SEQ ID NO: 49)** | TTGTCAAGTCTCTGCTGGCCCAGCCAAACCCTG TCTGACAACCTCTTGGTGAACCTTAGTACCTAA AAGGAAATCTCACCCTATCCC |
| **TP53_PCR primer 1 (SEQ ID NO: 50)** | GCATAAAACAAGTCTTGGTGGATC |
| **TP53_PCR primer 2 (SEQ ID NO: 51)** | TTGTCAAGTCTCTGCTGGCC |

**[Table 15]**

| **Gene: ACTB** | |
|---|---|
| **Sequence Type** | Sequence Information (5' → 3') |
| **ACTB_target promoter probe (SEQ ID NO: 52)** | [Phosphate]GTCCTCACTGCCTGTCCCCTTC CCCCTATAGTGAGTCGTATTAATTTCGCGACAA CACGCGAAATTAATACGACTCACTATAGGG |
| **ACTB_target reporter probe (SEQ ID NO: 53)** | GTATGTGGGAGCCCACACTCTACTCGACAGATA CGAATATCTGGACCCGACCGTCTCCCACATACG TGTGGGGAGCTGTCACATCCAGG |
| **ACTB_non-target promoter probe (SEQ ID NO: 54)** | [Phosphate]GTCCTCACTGCCTGTCCCCTTC CCCCTATAGTGAGTCGTATTAATTTCGCGACAA CACGCGAAATTAATACGACTCACTATAGGG |
| **ACTB_non-target reporter probe (SEQ ID NO: 55)** | GTATGTGGGAGCCCACACTCTACTCGACAGATA CGAATATCTGGACCCGACCGTCTCCCACATACG TGTGGGGAGCTGTCACATCCAGT |
| **Downstream double strand recombination-interfering DNA 1, ACTB (SEQ ID NO: 56)** | TGTCTTTCCTGCCTGAGCTGACCTGGGCAGGTC GGCTGTGGG |
| **Downstream double strand recombination-interfering DNA 2, ACTB (SEQ ID NO: 57)** | TGTCTTTCCTGCCTGAGCTGACCTGGGCAGGTC GGCTGTGGGGTCCTGTGGT |
| **Upstream double strand recombination-interfering DNA, ACTB (SEQ ID NO: 58)** | TATGATGATCTGGATCCACCAAGACTTGTTTTA TGC |
| **ACTB_target sequence (SEQ ID NO: 59)** | CGCCGATCCACACGGAGTACTTGCGCTCAGGAG GAGCAATGATCTGAGGAGGGAAGGGGACAGGCA GTGAGGACCCTGGATGTGACAGCTCCCCACACA CCACAGGACCCCACAGCCGACCTGCCCAGGTCA GCTCAGGCAGGAAAGACA |
| **ACTB_non-target sequence (SEQ ID NO: 60)** | CGCCGATCCACACGGAGTACTTGCGCTCAGGAG GAGCAATGATCTGAGGAGGGAAGGGGACAGGCA GTGAGGACACTGGATGTGACAGCTCCCCACACA CCACAGGACCCCACAGCCGACCTGCCCAGGTCA GCTCAGGCAGGAAAGACA |
| **ACTB_target PCR primer 1 (SEQ ID NO: 61)** | CGCCGATCCACACGGAGTACTTGCGCTCAGGAG GAGCAATGATCTGAGGAGGGAAGGGGACAGGCA GTGAGGACCCTGGATGTG |
| **ACTB_target PCR primer 2 (SEQ ID NO: 62)** | TGTCTTTCCTGCCTGAGCTGACCTGGGCAGGTC GGCTGTGGGGTCCTGTGGTGTGTGGGGAGCTGT CACATCCAGGGTCCTCACTG |
| **ACTB_non-target PCR primer 1 (SEQ ID NO: 63)** | GCATAAAACAAGTCTTGGTGGATCCAGATCATC ATATACAAGAGATGAAATCCTCCAGGGTGTGGG ATAGGGTGAGATTTCCTTTTAG |
| **ACTB_non-target PCR primer 2 (SEQ ID NO: 64)** | TTGTCAAGTCTCTGCTGGCCCAGCCAAACCCTG TCTGACAACCTCTTGGTGAACCTTAGTACCTAA AAGGAAATCTCACCCTATCCC |
| **ACTB_PCR primer 1 (SEQ ID NO: 65)** | GCATAAAACAAGTCTTGGTGGATC |
| **ACTB_PCR primer 2 (SEQ ID NO: 66)** | TTGTCAAGTCTCTGCTGGCC |

Meanwhile, the process of performing the SENSR reaction for discriminating and detecting human genome SNPs is shown below. Specifically, the PCR reaction products in Tables 13 and 14 were mixed with double strand recombination-interfering DNA and a SENSR probe set, and were reacted under the conditions in Table 16 below.

Further, the resulting reaction product was reacted at 98°C for 5 minutes to denature double-stranded DNA. Then, the reaction product was cooled to 37°C, and the SENSR reactant in Table 17 was added and reacted at 37°C for 15 minutes.

In addition, in order to measure the fluorescence resulting from the above reaction, 20 µl of the reaction product was dispensed into a 384-well clear flat-bottom black microplate. Then, the microplate was placed in a microplate reader (Hidex Sense), and the fluorescence was measured after setting the absorption/emission wavelength for the fluorescent dye (absorption: 616 nm / emission: 665 nm for malachite green).

**[Table 16]**

| **Reagent** | **Concentration** | **Amount** |
|---|---|---|
| **Purified PCR resultant** | Variable | 6 **µℓ** |
| **Downstream double strand recombination-interfering DNA** | 20 mM | 3 **µℓ** |
| **Upstream double strand recombination-interfering DNA** | 20 mM | 3 **µℓ** |
| **Promoter probe (PP)** | 10 **µ**M | 0.6 **µℓ** |
| **Reporter probe (RP)** | 10 **µ**M | 1.32 **µℓ** |
| **SENSR buffer** | 10X | 1.08 **µℓ** |
| **RNase-free water** | | 3 **µℓ** |
| Total | | 18 **µℓ** |

**[Table 17]**

| **Reagent** | **Concentration** | **Amount** |
|---|---|---|
| **Sample (Resultant i)** | - | 9 **µℓ** |
| **SENSR buffer** | 10X | 3 **µℓ** |
| **NTPs** | 25 mM each | 3 **µℓ** |
| **ATP** | 1 M | 0.3 **µℓ** |
| **ET-SSB** | 500 ng/**Mℓ** | 1 **µℓ** |
| **RNase Inhibitor** | 40 u/**Mℓ** | 1 **µℓ** |
| **SplintR ligase** | 25 u/**Mℓ** | 3 **µℓ** |
| **T7 RNA polymerase** | 50 **Mℓ** | 1.5 **µℓ** |
| **Malachite green** | 320 **µ**M | 1.5 **µℓ** |
| **RNase-free water** | | Up to 30 **µℓ** |
| **Total** | | 30 **µℓ** |

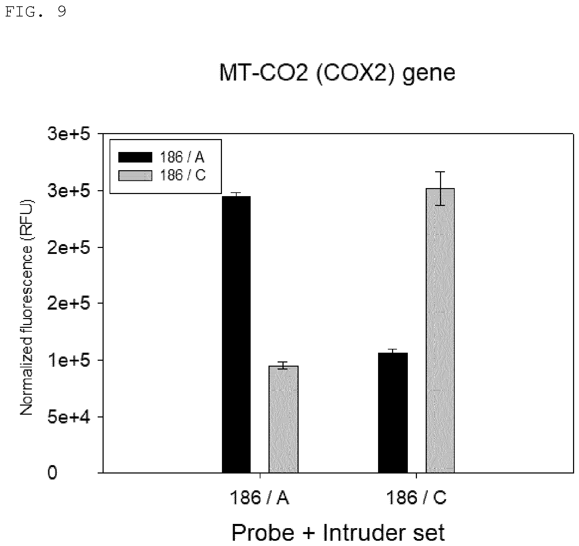

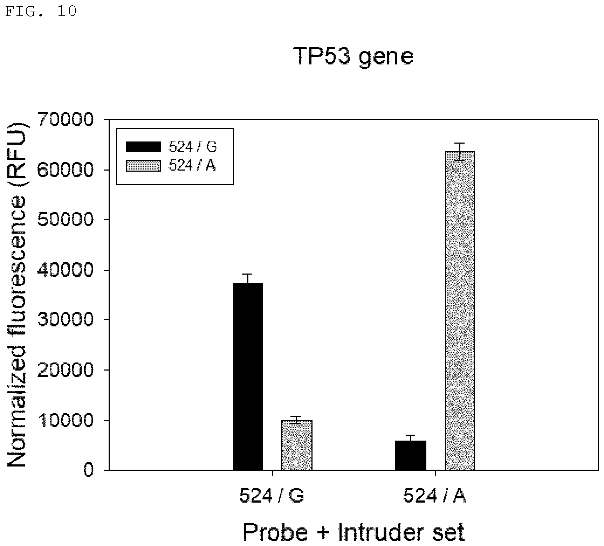

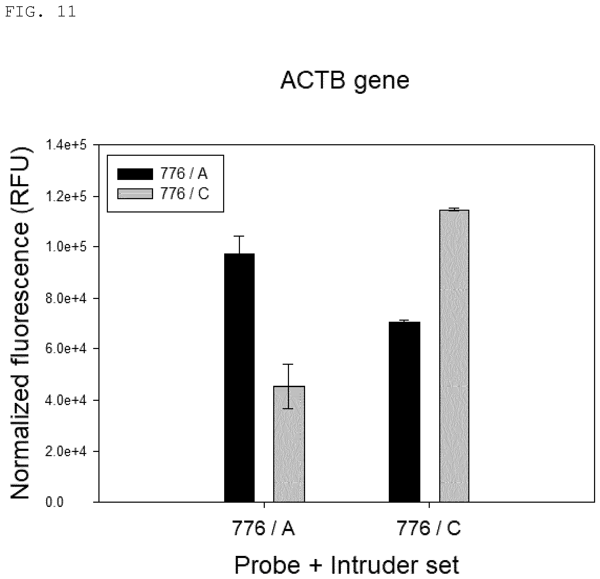

As a result of the experiment, as shown in FIGS. 9 to 11, it was confirmed that each of the three SNPs present in the human genome could be easily discriminated and detected from the PCR amplicon only by the reaction of about 20 minutes. This result implies that the method according to the present invention is able to detect not only viruses but also SNP regions present in the human genome.

## Claims

1. A composition for detecting a target nucleic acid comprising:
an isothermal one-pot reaction SENSR probe set for detecting a target nucleic acid including a first probe and a second probe; and
at least one nucleic acid for interfering with double strand recombination of target gene,
wherein the first probe is a promoter probe (PP) having a structure of the following general formula (I);
3'-X-Y-5' (I)
in the general formula (I) above,
X is a stem-loop structure portion containing a promoter sequence recognizable by an RNA polymerase; Y is an upstream hybridization sequence (UHS) portion having a hybridization sequence complementary to a target nucleic acid sequence; the target nucleic acid sequence is DNA or RNA; and X and Y are deoxyribonucleotides;
the second probe is a reporter probe (RP) having a structure of the following general formula II;
3'-Y'-Z-5' (II)
in the general formula (II) above,
Y' is a downstream hybridization sequence (DHS) portion having a hybridization sequence complementary to the target nucleic acid sequence; Z is an aptamer sequence portion with an interactive labeling system comprising one label or a plurality of labels generating a detectable signal; the target nucleic acid sequence is DNA or RNA; and Y' and Z are deoxyribonucleotides; and
the nucleic acid for interfering with double strand recombination of target gene is a sequence complementary to the target nucleic acid sequence at the 3' end or 5' end of a complementary binding region of the SENSR probe set.

2. The composition of claim 1, wherein the target nucleic acid is any one selected from genes of viruses, harmful bacteria, and animals, including humans.

3. The composition of claim 1, wherein the genes of animals, including humans, are single nucleotide polymorphism (SNP) genes.

4. The composition of claim 1, wherein one or two nucleic acids for interfering with double strand recombination of target gene are present.

5. The composition of claim 4, wherein when the two nucleic acids for interfering with double strand recombination of target gene are present, the nucleic acids are sequences complementary to the target nucleic acid sequence at the 3' end or the 5' end, respectively.

6. The composition of claim 1, wherein the nucleic acid for interfering with double strand recombination of target gene is a sequence that binds complementary to the target gene at a position 0 to 24 nucleotide sequences away from the complementary binding region of the SENSR probe set.

7. The composition of claim 1, wherein the nucleic acid for interfering with double strand recombination of target gene has 8 or more nucleotide sequences.

8. The composition of claim 1, wherein the label is any one selected from the group consisting of a chemical label, an enzyme label, a radioactive label, a fluorescent label, a luminescent label, a chemiluminescent label, and a metal label.

9. The composition of claim 1, wherein the isothermal one-pot reaction is performed simultaneously in one vessel, without a separate amplification reaction, unified by any one specified temperature in the range of 15°C to 50°C.

10. A composition for detecting a target gene comprising: an isothermal one-pot reaction SENSR first probe set for detecting a target nucleic acid, including a first probe and a second probe; an isothermal one-pot reaction SENSR second probe set for detecting a target nucleic acid mutation, including a third probe and a fourth probe; and at least one nucleic acid for interfering with double strand recombination of target gene,
wherein the first probe is a promoter probe (PP) having a structure of the following general formula (I);
3'-X-Y-5' (I)
in the general formula (I) above,
X is a stem-loop structure portion containing a promoter sequence recognizable by an RNA polymerase; Y is an upstream hybridization sequence (UHS) portion having a hybridization sequence complementary to a target nucleic acid sequence;
the target nucleic acid sequence is DNA or RNA; and X and Y are deoxyribonucleotides;
the second probe is a reporter probe (RP) having a structure of the following general formula II;
3'-Y'-Z-5' (II)
in the general formula (II) above,
Y' is a downstream hybridization sequence (DHS) portion having a hybridization sequence complementary to the target nucleic acid sequence; Z is an aptamer sequence portion with an interactive labeling system comprising one label or a plurality of labels generating a detectable signal; the target nucleic acid sequence is DNA or RNA; and Y' and Z are deoxyribonucleotides; and
the third probe is a promoter probe (PP) having a structure of 3'-X"-Y"-5' (I); X" is a stem-loop structure portion containing a promoter sequence recognizable by an RNA polymerase; Y" is an upstream hybridization sequence (UHS) portion having a hybridization sequence complementary to the target nucleic acid sequence; the target nucleic acid sequence is DNA or RNA; and X" and Y" are deoxyribonucleotides;
the fourth probe is a reporter probe (RP) having a structure of 3'-Y‴-Z‴-5' (II); Y‴ is a downstream hybridization sequence (DHS) portion having a hybridization sequence complementary to a target SNP nucleic acid sequence; Z‴ is an aptamer sequence portion having an interactive labeling system comprising one label or a plurality of labels that generate a detectable signal; the target nucleic acid sequence is DNA or RNA; and Y‴ and Z‴ are deoxyribonucleotides; and
the at least one nucleic acid for interfering with double strand recombination of target gene is a sequence complementary to the target nucleic acid sequence at the 3' end or at the 5' end of a complementary binding region of the SENSR first probe set or the SENSR second probe set.

11. The composition of claim 10, wherein the composition is used to detect mutations in genes.

12. The composition of claim 11, wherein the mutation includes mutations in genes of viruses, harmful bacteria, and animals, including humans.

13. The composition of claim 12, wherein the mutations in genes of animals, including humans, are mutations in single nucleotide polymorphism (SNP) genes.

14. A kit for detecting a target gene comprising:
the composition according to any one of claims 1 to 13;
a ligation agent,
RNA polymerase, and
an isothermal one-pot reaction buffer.

15. The kit of claim 14, wherein the ligation agent is any one selected from the group consisting of SplintR ligase, bacteriophage T4 ligase, *E. coli* ligase, Afu ligase, Tag ligase, Tfl ligase, Mth ligase, Tth ligase, Tth HB8 ligase, *Thermus* species AK16D ligase, Ape ligase, LigTk ligase, Aae ligase, Rm ligase, Pfu ligase, ribozyme and variants thereof.

16. The kit of claim 14, wherein the RNA polymerase is selected from the group consisting of bacteriophage T7 RNA polymerase, bacteriophage T3 polymerase, bacteriophage RNA polymerase, bacteriophage ΦII polymerase, *Salmonella* bacteriophage sp6 polymerase, *Pseudomonas* bacteriophage gh-1 polymerase, *E. coli* RNA polymerase holoenzyme, *E. coli* RNA polymerase core enzyme, human RNA polymerase I, human RNA polymerase II, human RNA polymerase III, human mitochondrial RNA polymerase and variants thereof.

17. The kit of claim 14, wherein the isothermal one-pot reaction buffer is any one selected from the group consisting of Tris-HCl, MgCl₂, NTPs, NaCl, and ET-SSB (Extreme Thermostable Single-Stranded DNA Binding Protein).

18. A method for detecting a target gene, comprising:
(a) separating a double strand of the target gene at a high temperature of 80 to 100°C;
(b) cooling a reaction product in (a) to an isothermal temperature of 15 to 50°C, and then performing an isothermal one-pot reaction by adding the composition according to any one of claims 1 to 8; and
(c) detecting signal generation.

19. A composition for detecting type 2 severe respiratory syndrome virus, comprising: an isothermal one-pot reaction SENSR probe set for type 2 severe respiratory syndrome virus, including a promoter probe of SEQ NO: 1 and a reporter probe of SEQ NO: 2; and a nucleic acid for interfering with double strand recombination of SEQ NO: 6, SEQ NO: 7, or both.

20. A composition for detecting type 2 severe respiratory syndrome virus omicron variants, comprising:
an isothermal one-pot reaction SENSR probe set for type 2 severe respiratory syndrome virus omicron variants, including a promoter probe of SEQ NO: 8 and a reporter probe of SEQ NO: 9; and
a nucleic acid for interfering with double strand recombination of SEQ NO: 10, SEQ NO: 11, or both.

21. A composition for simultaneous detection of type 2 severe respiratory syndrome virus and omicron variants thereof, comprising: an isothermal one-pot reaction SENSR probe set for type 2 severe respiratory syndrome virus omicron variants, including a promoter probe of SEQ NO: 8 and a reporter probe of SEQ NO: 9 and SEQ NO: 15; and a nucleic acid for interfering with double strand recombination of SEQ NO: 10, SEQ NO: 16, or both.

22. The composition of claim 21, further comprising: a nucleic acid for interfering with double strand recombination of SEQ NO: 17.

23. A kit for detecting type 2 severe respiratory syndrome virus or omicron variants thereof, comprising: the composition according to any one of claims 19 to 22; a ligation agent; a polymerase; and an isothermal one-pot reaction buffer.

24. A diagnostic method for detecting target SARS-CoV-2 or omicron variants thereof, comprising:
(a) separating a double strand of a target gene at a high temperature of 80 to 100°C;
(b) cooling a reaction product in (a) to an isothermal temperature of 15 to 50°C and then performing an isothermal one-pot reaction by adding the composition according to any one of claims 19 to 22; and
(c) confirming luminescence.

25. A composition for detecting a target nucleic acid comprising a nucleic acid for interfering with double strand recombination of target gene for an isothermal amplification reaction, wherein the nucleic acid for interfering with double strand recombination of target gene is a sequence that binds complementarily to the target gene at a position 0 to 24 nucleotide sequences away from a complementary binding region of the probe set.

26. The composition of claim 25, wherein the nucleic acid for interfering with double strand recombination of target gene is located at the 3' end or the 5' end or both of the complementary binding region of the probe set.

27. The composition of claim 25, wherein the nucleic acid for interfering with double strand recombination has 8 to 60 nucleic acid sequences.

28. A method for providing information for single nucleotide polymorphism (SNP)-based disease diagnosis, comprising:
(a) separating a double strand of a target gene at a high temperature of 80 to 100°C;
(b) cooling a reaction product in (a) to an isothermal temperature of 15 to 50°C and then performing an isothermal one-pot reaction by adding the composition according to claim 8;
(c) detecting signal generation; and
(d) determining the onset of a genetic disease when a mutation at the level of single nucleotide polymorphism (SNP) in the gene is confirmed.
